# EUROPÄISCHE PATENTSCHRIFT

(11) **EP 3 934 562 B1**
(45) Veröffentlichungstag und Bekanntmachung des Hinweises auf die Patenterteilung: **08.03.2023**
(21) Anmeldenummer: 20709545.6
(22) Anmeldetag: 04.03.2020
(51) Int. Cl.: A61C 5/00

(54) **KLEBERETAINER**
BONDED RETAINER
SYSTÈME DE BLOCAGE ADHÉSIF

(30) Priorität: 05.03.2019 DE 102019105501
(43) Veröffentlichungstag der Anmeldung: 12.01.2022
(73) Patentinhaber: CA-Digital GmbH, 40724 Hilden (DE)
(72) Erfinder: GAUGGEL, Markus, 40545 Düsseldorf (DE)
(74) Vertreter: Bauer, Dirk
(86) Internationale Anmeldenummer: PCT/EP2020/055733
(87) Internationale Veröffentlichungsnummer: WO 2020/178353

(56) Entgegenhaltungen:
- WO-A1-2004/028394
- JP-A- 2015 096 243

## Beschreibung

Die Erfindung betrifft einen Kleberetainer zur Stabilisierung einer Mehrzahl von Zähnen in einem Kiefer, mit einer Kontaktfläche, die in einer Längsrichtung des Kleberetainers global einer parabelförmigen Krümmung des Kiefers und lokal einer zungenseitigen Oberflächenkontur der Zähne folgt, und mit einer zungenseitig parallel zu der Kontaktfläche verlaufenden Lingualfläche.

Retainer sind Vorrichtungen zur dauerhaften Stabilisierung der Zähne eines Patienten zum Abschluss einer kieferorthopädischen Behandlung, insbesondere zur Korrektur einer Fehlstellung. Durch den Retainer kann der Kieferknochen mit den Zahnwurzeln in der kieferorthopädisch neu geschaffenen Position verwachsen. Die Gefahr eines Rezidivs kann so vermindert werden. Ein Kleberetainer (auch: "fester Retainer") wird mit der zungenseitigen Oberfläche der Zähne verklebt. Ein drahtförmiger Kleberetainer der vorgenannten Art ist bekannt aus DE 10 2013 204 359 A1, DE 10 2016 109007 A1 schlägt vor, einen Kleberetainer der vorgenannten Art aus PEEK durch 3D-Druck herzustellen. Auch Kleberetainer aus mehreren verlitzten oder geflochtenen Einzeldrähten sind allgemein bekannt.

### Aufgabe

Der Erfindung liegt die Aufgabe zugrunde, die Stabilität des Retainers zu verbessern.

### Lösung

Ausgehend von dem bekannten Kleberetainer werden nach der Erfindung quer zur Längsrichtung angeordnete Retentionen aus der Lingualfläche vorgeschlagen. Beim Verkleben dringt der Kleber formschlüssig zwischen die Retentionen und vermeidet ein Gleiten des Kleberetainers in seiner Längsrichtung in der Klebestelle.

Die Retentionen können auf die Lingualfläche aufgesetzt oder aus dieser herausgearbeitet sein.

Vorzugsweise weisen an einem erfindungsgemäßen Kleberetainer die Retentionen lokal eine Höhe von mehr als 0,05 mm auf. Mit mindestens fünf Retentionen ist der Kleberetainer dann gegen Gleiten in der Klebestelle ausreichend gesichert. Vorzugsweise weisen die Retentionen eine Höhe von mehr als 0,1 *mm,* weiter vorzugsweise mehr als 0,15 *mm* auf. Mit steigender Höhe der Retentionen kann die Anzahl der Retentionen je Klebestelle vermindert werden.

Vorzugsweise ist an einem erfindungsgemäßen Kleberetainer die Kontaktfläche sägeblattartig profiliert. Der regelmäßige Abstand der Einkerbungen bewirkt eine gleichmäßig feste Verankerung des Kleberetainers in der Klebestelle.

Vorzugsweise wird ein erfindungsgemäßer Kleberetainer drahtförmig derart aus einer Platte geschnitten, dass die Kontaktfläche, die Lingualfläche und zwei zueinander parallel verlaufende Ausschnitte einer Oberfläche der Platte einen parallelogrammförmigen Querschnitt des Kleberetainers bilden.

Vorzugsweise besteht die Platte, aus der ein solcher erfindungsgemäßer Kleberetainer geschnitten wird, aus einem Formgedächtnismaterial. Ein aus einem solchen Material geschnittener Retainer nimmt nach Belastung zuverlässig wieder die ursprünglich geschnittene Form ein. Vorzugsweise besteht die Platte aus einer Nickel-Titan-Legierung, weiter vorzugsweise aus Nitinol. Diese Legierungen sind für die Herstellung eines erfindungsgemäßen Kleberetainers besonders geeignet.

Alternativ kann ein erfindungsgemäßer Retainer aus einem Titan- oder Stahlblech geschnitten oder aus einem Titan- oder Stahldraht gebogen werden.

Vorzugsweise weist der Querschnitt eines erfindungsgemäßen Kleberetainers Kantenlängen von nicht mehr als 0,7 *mm* auf. Ein solcher Retainer wird als besonders wenig störend empfunden. Vorzugsweise weist der Querschnitt Kantenlängen von nicht mehr als 0,5 *mm,* weiter vorzugsweise nicht mehr als 0,3 mm auf. Mit wachsendem Querschnitt steigt die mechanische Stabilität des Retainers.

Alternativ vorzugsweise kann ein erfindungsgemäßer Kleberetainer durch 3D-Druck hergestellt sein. Im 3D-Druck wird der Kleberetainer schichtweise dreidimensional aufgebaut.

Vorzugsweise wird ein solcher erfindungsgemäßer Kleberetainer aus einem thermoplastischen Kunststoff hergestellt. Thermoplastische Kunststoffe lassen sich besonders einfach im 3D-Druck verarbeiten oder auch fräsen. Insbesondere kommt Polyetheretherketon (PEEK) zur Herstellung eines erfindungsgemäßen Kleberetainers in Betracht. Alternativ können auch Polyetherketon, Fluorkunststoffe wie Polyvinylidenfluorid (PVFD) und Polyoxymethylen (POM) oder Polymide wie Polyetherimid (PEI) zum Einsatz kommen. Weiter alternativ kann ein solcher erfindungsgemäßer Kleberetainer aus einem Kunstharz, einer Keramik oder aus Metall hergestellt werden.

Vorzugsweise sind an einem solchen erfindungsgemäßen Kleberetainer die Retentionen pilzförmig ausgebildet. Die Retentionen verankern den Kleberetainer dann besonders gut in der Klebestelle.

Weiter alternativ kann ein erfindungsgemäßer Retainer aus Metall gefräst werden.

Vorzugsweise ist eine Oberfläche eines erfindungsgemäßen Kleberetainers poliert. Ein solcher Retainer wird als besonders wenig störend empfunden. Vorzugsweise ist die Oberfläche mittels Elektropolitur oder Plasmapolitur behandelt. Diese Verfahren sind zum Polieren von Nickel-Titan-Legierungen besonders geeignet.

### Ausführungsbeispiele

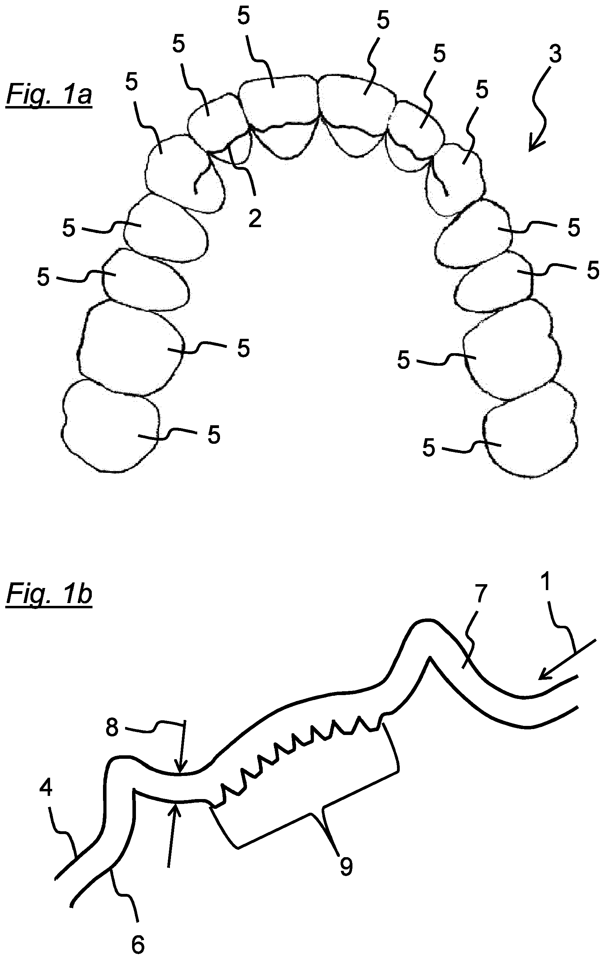

Die Erfindung wird nachfolgend anhand von Ausführungsbeispielen erläutert. Es zeigen
Fig. 1a einen ersten erfindungsgemäßen Kleberetainer,
Fig. 1b ein Detail des ersten erfindungsgemäßen Kleberetainers und
Fig. 1c einen Abschnitt des Details,
Fig. 2 und 3 Abschnitte eines zweiten und eines dritten erfindungsgemäßen Kleberetainers,
Fig. 4a einen vierten erfindungsgemäßen Kleberetainer,
Fig. 4b ein Detail des vierten erfindungsgemäßen Kleberetainers und
Fig. 4c einen Schnitt durch das Detail sowie
Fig. 5 einen Schnitt durch einen fünften erfindungsgemäßen Kleberetainer,
Fig. 6a einen sechsten erfindungsgemäßen Kleberetainer und
Fig. 6b ein Detail des sechsten erfindungsgemäßen Kleberetainers.

Die Längsrichtung 1 des in den Zeichnungsfiguren 1a bis 1c gezeigten ersten erfindungsgemäßen drahtförmigen Kleberetainers 2 folgt global einer parabelförmigen Krümmung des Kiefers 3 eines Patienten. Der Kleberetainer 2 ist aus einer nicht dargestellten Nitinol-Platte mit einer Dicke von 0,4 mm geschnitten und weist einen im Wesentlichen rechteckigen Querschnitt auf. Nach dem Schneiden ist der Kleberetainer 2 mit einer Plasmapolitur behandelt.

Der Kleberetainer 2 weist eine Kontaktfläche 4 auf, die lokal einer zungenseitigen Oberflächenkontur der Zähne 5 folgt und in der kieferorthopädischen Anwendung an den Zähnen 5 anliegt. Im Wesentlichen parallel zu der Kontaktfläche 4 verläuft eine der Zunge des Patienten zugewandte Lingualfläche 6. Ausschnitte 7 aus den Oberflächen der Platte schließen den Querschnitt. Die Ausschnitte 7 weisen im Querschnitt eine Kantenlänge 8 von mindestens 0,4 mm auf.

Die Kontaktfläche 4 weist sägeblattartig profilierte Abschnitte 9 auf mit trapezförmigen Retentionen 10 mit quer zu der Längsrichtung 1 verlaufenden Einkerbungen 11 und mit einer Höhe 12 von ca. 0,2 *mm.* In der kieferorthopädischen Anwendung werden die Abschnitte 9 in einen Nanohybridkompositkleber eingebettet, der zwischen die Retentionen 10 in die Einkerbungen 11 eindringt. Nach dem Aushärten ist der Kleberetainer 2 in seiner Längsrichtung 1 in den Klebestellen durch Formschluss gegen Gleiten gesichert.

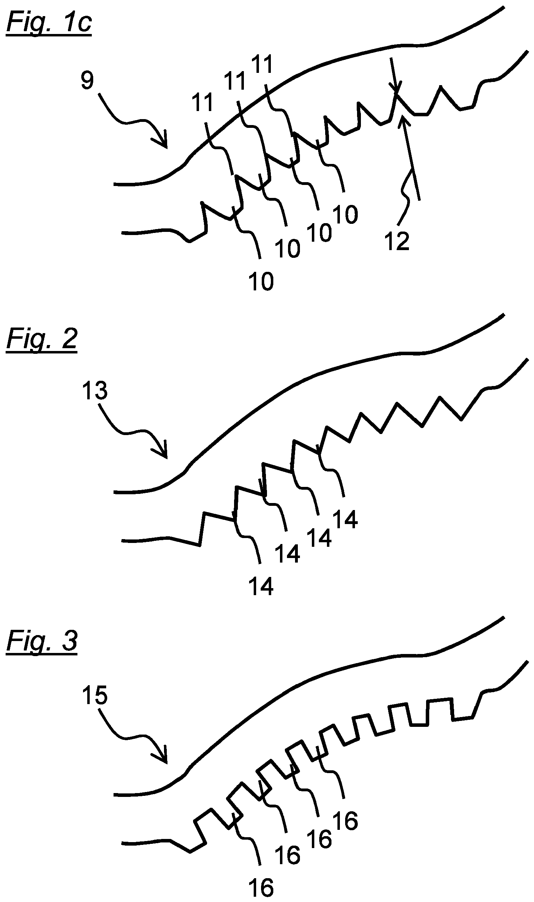

Figur 2 zeigt einen Abschnitt 13 eines zweiten erfindungsgemäßen Kleberetainers mit dreieckigen Retentionen 14. Figur 3 zeigt einen Abschnitt 15 eines dritten erfindungsgemäßen Kleberetainers mit rechteckigen Retentionen 16 und entsprechend rechteckig profilierten Einkerbungen 17 zwischen den Retentionen.

Der zweite und der dritte Kleberetainer entsprechen bis auf die Abschnitte 13 und 15 dem ersten Kleberetainer 2 und sind daher nicht weiter dargestellt.

Der in der Zeichnungsfigur 4a gezeigte vierte erfindungsgemäße Kleberetainer 18 ist im 3D-Druck aus Polyetheretherketon (PEEK) hergestellt. Eine Längsrichtung 19 des vierten Kleberetainers 18 folgt wiederum global einer parabelförmigen Krümmung des Kiefers 20 eines Patienten. Eine Kontaktfläche 21 des vierten Kleberetainers 18 folgt lokal einer zungenseitigen Oberflächenkontur der Zähne 22 und liegt in der kieferorthopädischen Anwendung an den Zähnen 22 an.

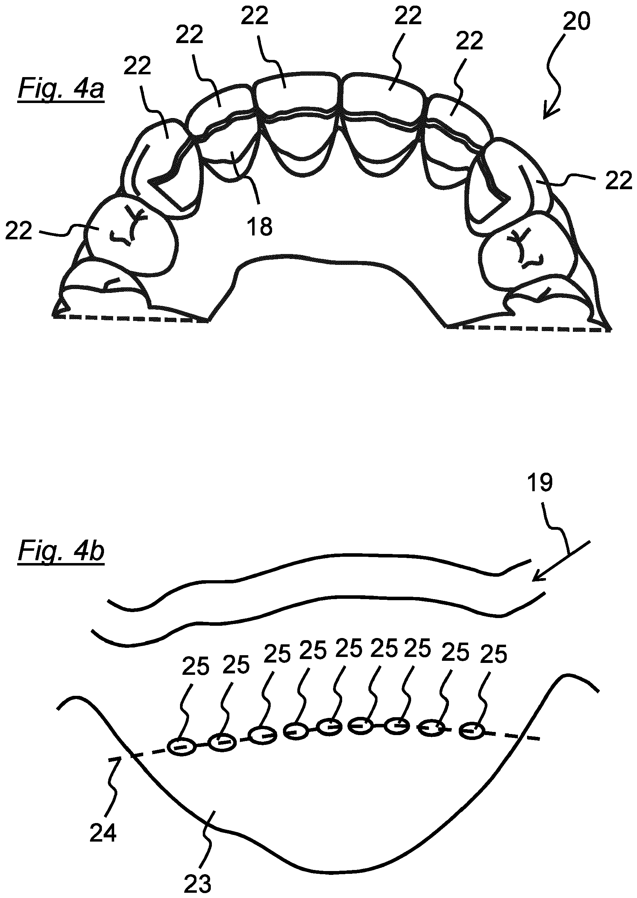

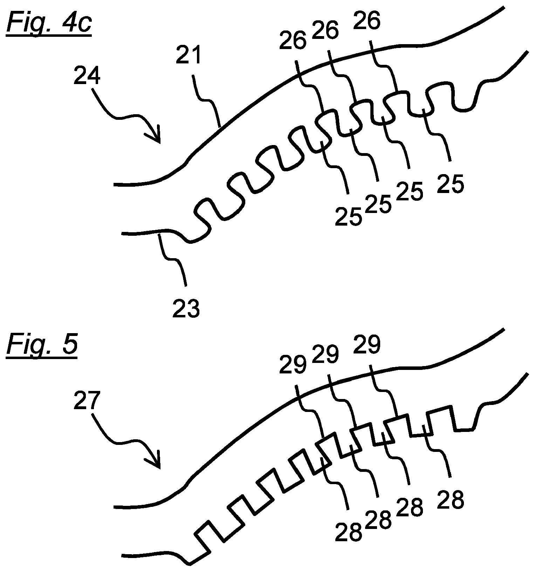

Im Wesentlichen parallel zu der Kontaktfläche 21 verläuft eine der Zunge des Patienten zugewandte Lingualfläche 23 mit im Detail in Figur 4b und im Schnitt 24 in Figur 4c dargestellten pilzförmigen Retentionen 25 und entsprechenden Einkerbungen 20 zwischen den Retentionen 25. Figur 5 zeigt einen entsprechenden Schnitt 27 durch einen fünften erfindungsgemäßen Kleberetainers aus PEEK mit keilförmigen Retentionen 28 und entsprechenden Einkerbungen 29 zwischen den Retentionen 28. Der fünfte Kleberetainer entspricht bis auf die Form der Retentionen dem vierten Kleberetainer 18 und ist daher nicht weiter dargestellt.

Der in der Zeichnungsfigr 6a gezeigte sechste erfindungsgemäße Kleberetainer ist gleichfalls im 3D-Druck aus Stahl hergestellt. Eine Längsrichtung 31 des sechsten Kleberetainers 30 folgt auch hier global einer parabelförmigen Krümmung des Kiefers 32 des Patienten. Eine Kontaktfläche 33 des sechsten Kleberetainers 30 folgt lokal einer zungenseitigen Oberflächenkontur der Zähne 34 und liegt in der kieferorthopädischen Anwendung an den Zähnen 34 an.

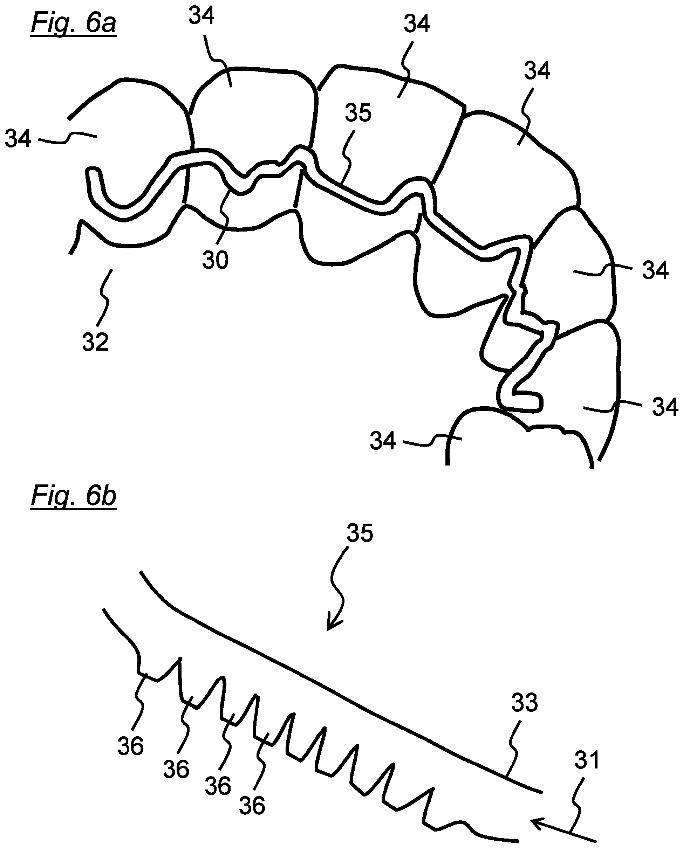

In einem in Figur 6b im Detail dargestellten, im wesentlichen geraden Abschnitt 35 weist der sechste Kleberetainer 30 entsprechende dem ersten Kleberetainer 2 trapezförmige Retentionen 36 auf, die den sechsten Kleberetainer 30 in der kieferorthopädischen Anwendung in einer nicht dargestellten Klebestelle verankern.

### In den Figuren sind

- 1: Längsrichtung
- 2: Kleberetainer
- 3: Kiefer
- 4: Kontaktfläche
- 5: Zahn
- 6: Lingualfläche
- 7: Ausschnitt
- 8: Kantenlänge
- 9: Abschnitt
- 10: Retention
- 11: Einkerbung
- 12: Tiefe
- 13: Abschnitt
- 14: Retention
- 15: Abschnitt
- 16: Retention
- 17: Einkerbung
- 18: Kleberetainer
- 19: Längsrichtung
- 20: Kiefer
- 21: Kontaktfläche
- 22: Zahn
- 23: Lingualfläche
- 24: Schnitt
- 25: Retention
- 26: Einkerbung
- 27: Schnitt
- 28: Retention
- 29: Einkerbung
- 30: Kleberetainer
- 31: Längsrichtung
- 32: Kiefer
- 33: Kontaktfläche
- 34: Zahn
- 35: Abschnitt
- 36: Retention

## Patentansprüche

1. Kleberetainer (2, 18, 30) zur Stabilisierung einer Mehrzahl von Zähnen (5, 22, 34) in einem Kiefer (3, 20, 32), mit einer Kontaktfläche (4, 21, 33), die in einer Längsrichtung (1, 19) des Kleberetainers (2, 18, 30) global einer parabelförmigen Krümmung des Kiefers (3, 20, 32) und lokal einer zungenseitigen Oberflächenkontur der Zähne (5, 22, 34) folgt, und mit einer zungenseitig parallel zu der Kontaktfläche (4, 21, 33) verlaufenden Lingualfläche (6, 23), ***gekennzeichnet durch*** quer zur Längsrichtung (1, 19) angeordnete Retentionen (10, 14, 16, 25, 28, 36) aus der Lingualfläche (6, 23).

2. Kleberetainer (2, 18, 30) nach dem vorgenannten Anspruch, ***dadurch gekennzeichnet, dass*** die Retentionen (10, 14, 16, 25, 28, 36) lokal eine Höhe (12) von mindestens 0,05 *mm,* vorzugsweise mindestens 0,1 *mm,* weiter vorzugsweise mindestens 0,15 mm aufweisen.

3. Kleberetainer (2) nach einem der vorgenannten Ansprüche, ***dadurch gekennzeichnet, dass*** die Kontaktfläche (4) sägeblattartig profiliert ist.

4. Kleberetainer (2) nach einem der vorgenannten Ansprüche, ***dadurch gekennzeichnet, dass*** der Kleberetainer (2) drahtförmig derart aus einer Platte geschnitten ist, dass die Kontaktfläche (4), die Lingualfläche (6) und zwei zueinander parallel verlaufende Ausschnitte (7) einer Oberfläche der Platte einen parallelogrammförmigen Querschnitt des Kleberetainers (2) bilden.

5. Kleberetainer (2) nach dem vorgenannten Anspruch, ***dadurch gekennzeichnet, dass*** die Platte aus einem Formgedächtnismaterial, vorzugsweise einer Nickel-Titan-Legierung, weiter vorzugsweise Nitinol besteht.

6. Kleberetainer (2) nach einem der Ansprüche 4 oder 5, ***dadurch gekennzeichnet, dass*** der Querschnitt Kantenlängen (8) von nicht mehr als *0,7 mm,* vorzugsweise nicht mehr als 0,5 *mm,* weiter vorzugsweise nicht mehr als 0,3 mm aufweist.

7. Kleberetainer (18, 30) nach einem der Ansprüche 1 bis 3, ***dadurch gekennzeichnet, dass*** der Kleberetainer (18, 30) durch 3D-Druck hergestellt ist.

8. Kleberetainer (18) nach dem vorgenannten Anspruch, ***dadurch gekennzeichnet, dass*** der Kleberetainer (18) aus einem thermoplastischen Kunststoff, vorzugsweise aus Polyetheretherketon hergestellt ist.

9. Kleberetainer (18) nach einem der vorgenannten Ansprüche, ***dadurch gekennzeichnet, dass*** die Retentionen pilzförmig ausgebildet sind.

10. Kleberetainer (2, 18, 30) nach einem der vorgenannten Ansprüche, ***dadurch gekennzeichnet,* dass** eine Oberfläche des Kleberetainers (2, 18, 30) poliert, vorzugsweise mittels Elektropolitur oder Plasmapolitur behandelt ist.

## Claims

1. A bonded retainer (2, 18, 30), configured to stabilize a plurality of teeth (5, 22, 34) in a jaw (3, 20, 32), the bonded retainer comprising:
a contact surface (4, 21, 33) that is aligned overall with a parabolic curvature of the jaw in a longitudinal direction (1, 19) of the bonded retainer (2, 18, 30) and that is aligned locally with a lingual surface contour of the teeth (5, 22, 34);
a lingual surface (6, 23) that extends lingually in parallel with the contact surface (4, 21, 33), and
retentions (10, 14, 16, 25, 28, 36) that extend from the lingual surface (6, 23) transversal to the longitudinal direction (1, 19).

2. The bonded retainer (2, 18, 30) according to claim 1, wherein the retentions (10, 14, 16, 25, 28, 36) have a local height (12) of at least 0,05 mm, advantageously at least 0.1 mm, further advantageously at least 0.15 mm.

3. The bonded retainer (2) according to claim 1 or 2, wherein the contact surface (4) is configured with a saw tooth pattern profile.

4. The bonded retainer (2) according to one of the preceding claims, wherein the bonded retainer is wire shaped and cut from a sheet of material so that the contact surface (4), the lingual surface (6) and two parallel sections (7) of a surface of the sheet form a diamond shaped cross section of the bonded retainer (2).

5. The bonded retainer (2) according to claim 4, wherein the sheet is made from a shape memory material made from a nickel titanium alloy or from Nitinol.

6. The bonded retainer (2) according to claim 4 or 5, wherein the cross section has edge lengths of 0.7 mm at the most, 0.5 mm at the most or 0.3 mm at the most.

7. The bonded retainer (18, 30) according to one of the claims 1 through 3, wherein the bonded retainer (18, 30) is made by 3D-printing.

8. The bonded retainer (18) according to claim 7, wherein the bonded retainer (18) is made from a thermoplastic synthetic material or from polyetheretherketone.

9. The bonded retainer (18) according to one of the preceding claims, wherein the retentions are configured mushroom shaped.

10. The bonded retainer (2, 18, 30) according to one of the preceding claims, wherein a surface of the bonded retainer (2, 18, 30) is electropolished or plasma polished .

## Revendications

1. Dispositif de retenue de colle (2, 18, 30) pour stabiliser une pluralité de dents (5, 22, 34) dans une mâchoire (3, 20, 32), comprenant une face de contact (4, 21, 33) qui, dans un sens longitudinal (1, 19) du dispositif de retenue de colle (2, 18, 30), suit globalement une courbure parabolique de la mâchoire (3, 20, 32) et localement un contour de surface côté langue des dents (5, 22, 34), et comprenant une face linguale (6, 23) parallèle à la face de contact (4, 21, 33) côté langue, **caractérisé par** des rétentions (10, 14, 16, 25, 28, 36) à partir de la face linguale (6, 23) disposées transversalement au sens longitudinal (1, 19).

2. Dispositif de retenue de colle (2, 18, 30) selon la revendication précédente, **caractérisé en ce que** les rétentions (10, 14, 16, 25, 28, 36) présentent localement une hauteur (12) d'au moins 0,05 mm, de préférence d'au moins 0,1 mm, plus encore de préférence d'au moins 0,15 mm.

3. Dispositif de retenue de colle (2) selon l'une des revendications précédentes, **caractérisé en ce que** la face de contact (4) est profilée à la manière d'une lame de scie.

4. Dispositif de retenue de colle (2) selon l'une des revendications précédentes, **caractérisé en ce que** le dispositif de retenue de colle (2) est ainsi coupé en forme de fil à partir d'une plaque que la face de contact (4), la face linguale (6) et deux découpes (7) parallèles entre elles d'une surface de la plaque forment une section transversale en forme de parallélogramme du dispositif de retenue de colle (2).

5. Dispositif de retenue de colle (2) selon la revendication précédente, **caractérisé en ce que** la plaque est composée d'un matériau à mémoire de forme, de préférence un alliage nickel-titane, plus encore de préférence de nitinol.

6. Dispositif de retenue de colle (2) selon l'une des revendications 4 ou 5, **caractérisé en ce que** la section transversale présente des longueurs d'arête (8) ne faisant pas plus de 0,7 mm, de préférence pas plus de 0,5 mm, plus encore de préférence pas plus de 0,3 mm.

7. Dispositif de retenue de colle (18, 30) selon l'une des revendications 1 à 3, **caractérisé en ce que** le dispositif de retenue de colle (18, 30) est fabriqué par impression 3D.

8. Dispositif de retenue de colle (18) selon la revendication précédente, **caractérisé en ce que** le dispositif de retenue de colle (18) est fabriqué dans un plastique thermoplastique, de préférence en polyétheréthercétone.

9. Dispositif de retenue de colle (18) selon l'une des revendications précédentes, **caractérisé en ce que** les rétentions sont conçues en forme de champignon.

10. Dispositif de retenue de colle (2, 18, 30) selon l'une des revendications précédentes, **caractérisé en ce qu'**une surface du dispositif de retenue de colle (2, 18, 30) est polie, de préférence traitée par électropolissage ou polissage au plasma.
